(19) **Europäisches Patentamt / European Patent Office / Office européen des brevets**

(11) **EP 1 564 218 B1**

(12) **EUROPEAN PATENT SPECIFICATION**

(45) Date of publication and mention of the grant of the patent:
**14.02.2007 Bulletin 2007/07**

(51) Int Cl.:
*C07K 14/315* (2006.01)      *A61P 31/04* (2006.01)

(21) Application number: **04030053.5**

(22) Date of filing: **17.12.2004**

(54) **Peptide composition for oral cavity**

Peptizusammensetzung für die Mundhöhle

Composition peptidique pour la cavité buccale

(84) Designated Contracting States:
**AT BE BG CH CY CZ DE DK EE ES FI FR GB GR HU IE IS IT LI LT LU MC NL PL PT RO SE SI SK TR**

(30) Priority: **19.12.2003 JP 2003422472**

(43) Date of publication of application:
**17.08.2005 Bulletin 2005/33**

(73) Proprietor: **GC Corporation**
**Itabashi-ku,**
**Tokyo (JP)**

(72) Inventors:
• **Senpuku, Hidenobu**
**Yokohama-shi**
**Kanagawa-ken (JP)**
• **Masuzawa, Yumiko**
**GC Corporation**
**Tokyo (JP)**
• **Okada, Junichi**
**GC Corporation**
**Tokyo (JP)**

(74) Representative: **Müller-Boré & Partner**
**Patentanwälte**
**Grafinger Strasse 2**
**81671 München (DE)**

(56) References cited:
• **DEMUTH D R ET AL: "STREPTOCOCCAL-HOST INTERACTIONS STRUCTURAL AND FUNCTIONAL ANALYSIS OF A STREPTOCOCCUS-SANGUIS RECEPTOR FOR A HUMAN SALIVARY GLYCOPROTEIN" JOURNAL OF BIOLOGICAL CHEMISTRY, vol. 265, no. 13, 1990, pages 7120-7126, XP002330165 ISSN: 0021-9258**
• **SENPUKU H ET AL: "Identification of Streptococcus mutans PAc peptide motif binding with human MHC class II molecules (DRB1*0802, *1101, *1401 and *1405)" IMMUNOLOGY 1998 UNITED KINGDOM, vol. 95, no. 3, 1998, pages 322-330, XP008028150 ISSN: 0019-2805**
• **DEMUTH D R ET AL: "COMPARISON OF STREPTOCOCCUS-MUTANS AND STREPTOCOCCUS-SANGUIS RECEPTORS FOR HUMAN SALIVARY AGGLUTININ" MICROBIAL PATHOGENESIS, vol. 9, no. 3, 1990, pages 199-212, XP002330166 ISSN: 0882-4010**

SEQUENCE LISTING

<110> GC Corporation

<120> Composition to be applied in the mouth

<130> GCD1761

<160> 1

<170> Patent In version 3.1

<210> 1
<211> 13
<212> PRT
<213> Streptococcus sanguis

<400> 1

```
Asp Tyr Gln Ala Lys Leu Ala Ala Tyr Gln Lys Glu Leu
 1                     5                      10
```

SEQUENCE LISTING

<110> GC Corporation
<120> Composition to be applied in the mouth
<130> GCD1761
<160> 1
<170> Patent In version 3.1
<210> 1
<211> 13
<212> PRT
<213> Streptococcus sanguis
<400> 1

```
Asp Tyr Gln Ala Lys Leu Ala Ala Tyr Gln Lys Glu Leu
 1                     5                      10
```

**Description**

The present invention relates to a composition for an oral cavity having the effect for suppressing an initial adhesion of intraoral bacteria to a tooth and the periodontal tissue, that is, for preventing a formation of a biofilm in an oral cavity.

The biofilm is a film covering the surface of a microbial cell together with a substance or a precipitate secreted by bacteria itself when microorganisms such as bacteria or the like adhere to the surface of an object or an organism tissue and proliferate. In an oral cavity, plaque (bacterial plaque) is typical as the biofilm. The formation of the biofilm in an oral cavity is started by an organic ingredient such as protein or the like in saliva contacting with the enamel surface, a part of the organic ingredient being adhered to the enamel, and thereby an acquired pellicle being formed.

Then, when an intraoral bacteria in the saliva approaches near a tooth and contacts with the pellicle, the intraoral bacteria such as Streptococcus sanguis bacteria or the like is adsorbed to the pellicle, and a part of the bacteria adheres on the pellicle as it is. The adhered intraoral bacteria grows to the strong biofilm, by generating the adhesion of the other bacteria or the like. The adhesion of the other bacteria is generated with polysaccharide having tacky adhesiveness called as glucan or fructan made by using the nutrient or the like in the saliva. Then, the bacteria in the biofilm repeats the proliferation to produce acid. Thereby, dental caries, periodontal disease or the like is generated.

For solving these problems, various methods have been researched. For example, an antibacterial agent and its assistant agent were proposed. As the assistant agent, antibacterial and antifungal assistants were proposed as a composition for an oral cavity including a biofilm suppressing assistant such as xylitol, farnesol or the like (for example, refer to Japanese Patent Laid Open No. 2002-302404, 2002-284604). However, since these antibacterial and antifungal assistants have main objects to remove the biofilm, use of the antibacterial agent is indispensable. It is known that the antibacterial agent has not only a problem of generating resistant bacteria by itself but also a problem that indigenous bacteria existing in the oral cavity is sterilized, and thus there are limits to use the antibacterial agent as the composition for an oral cavity. Further, it has been tried that protease which is an enzyme decomposing protein, is added (for example, refer to Japanese Patent Laid Open No. 06-262165). However, since protease is an enzyme, there are problems that the time is required to have an effective result, and a beneficial protein contained in the saliva is also decomposed. Further, it has been also tried that a monoclonal antibody is added (for example, refer to Japanese Patent Laid Open No. 2002-114709). The monoclonal antibody suppresses glucosyltransferase, which is enzyme secreted by Streptococcus mutans bacteria when producing glucan. However, if the biofilm is once formed, the effect with respect to the proliferation of the intraoral bacterial is hardly obtained, and there is a problem in safety since the monoclonal antibody originating in the mouse is applied to the inside of an oral cavity of person.

Demuth DR et al., Journal of Biological Chemistry, Vol. 265, No. 13, 7120-7126, 1990, describes the nucleotide and deduced amino acid sequence of the *S. sangius* receptor SSP-5. Senpuku et al., Immunology, Vol. 95, No. 3, 322-330, 1998, describes the identification of antigenic peptide binding to major histocompatibility complex ciass II (HLA-DR) molecules in the A-region of a surface protein antigen of *Streptococcus mutans.* Demuth DR et al., Microbila Pathogenesis, Vol. 9, 199-211, 1990, describes the receptor (MSL-1) for salivary agglutinin from *S. mutans* KPSK2 and its comparison to the *S. Sanguis* M5 agglutinin receptor (SSP-5) and the P1 antigen.

The primary objective of the present invention is to provide a composition for an oral cavity, not giving resistance to the objective bacteria, having safety for a human body, and having effects in a short time. Further, this composition can effectively suppress the formation of the biofilm, but does not react to indigenous bacteria and saliva being not related to the formation of the biofilm in an oral cavity.

The earnest work was carried out and, as the result, it was found that a peptide comprising a specific amino acid sequence has the effect for suppressing the formation of the biofilm, by taking notice of the following point. That is, when the intraoral bacteria forming the biofilm is not sterilized but a substance suppressing an initial adhesion of bacteria to the oral cavity tissue is used, the formation of the biofilm can be suppressed. Then, the investigation was completed.

More particularly, it is known that Streptococcus bacteria in the intraoral bacteria has a strong initial adhesion to the oral cavity tissue such as a tooth surface covered with the pellicle. The biofilm is formed by adhesion of these bacteria to the pellicle on the tooth surface giving opportunities for further adhesion of other bacteria. Therefore, it is important to suppress the initial adhesion of the Streptococcus bacteria to the oral cavity tissue. For example, it was confirmed that the initial adhesion of the Streptococcus mutans depends on protein existing on the surface of bacteria, which is called as PAc (Protein Antigen Serotype C) with the molecular weight of about 190,000.

The most important part relating to the initial adhesion was researched, based on the A area of the PAc. Then, it was specified that a specific peptide part (hereinafter, it is said to as SSP-5(390-402)) in the protein is directly related to the initial adhesion of the Streptococcus intraoral bacteria to the tooth surface. This SSP-5(390-402) comprises a represented specific amino acid sequence number of the sequence listing and is one part of the protein on the surface of the Streptococcus sanguis bacteria. As a result, the following fact could be found out. That is, when this peptide comprising the specific amino acid sequence is previously applied to the inside of an oral cavity, such as the tooth surface or the like, this peptide can block the tooth surface from Streptococcus intraoral bacteria adhering thereon. The Streptococcus intraoral bacteria usually adhere on the tooth surface using this peptide part comprising an amino acid sequence but cannot adhere since the tooth surface has already been covered by this peptide part. Thus it is possible to effectively suppress the initial adhesion of bacteria in a short time.

In accordance with one aspect of the invention, there is provided a composition for the oral cavity, which contains a peptide of the amino acid sequence Asp Tyr Gln Ala Lys Leu Ala Ala Tyr Gln Lys Glu Leu.

The composition for the oral cavity according to the present invention is safe for an organism as compared with the conventional antibacterial agent since using an amino acid. When the composition according to the present invention is applied to the tooth surface or the like, the composition can effectively suppress the initial adhesion of bacteria in a short time, since the peptide previously covers the tooth surface for blocking, while bacteria usually adhere using the peptide. Further, the composition according to the present invention is an excellent composition for an oral cavity capable of effectively suppressing the formation of the biofilm without reacting to indigenous bacteria and saliva at all. These indigenous bacteria and saliva do not relate to the formation of the biofilm in an oral cavity.

For obtaining the peptide, used in the composition for an oral cavity according to the present invention, although it is not limited how such the amino acid sequence is obtained, an amino acid synthesizer can be preferably used in general. At the time of synthesizing the peptide, it is important that the peptide does not contain an excessive amino acid sequence in the above amino acid sequence. If there is a part of the excessive amino acid sequence, the original effect for suppressing the formation of the biofilm cannot be obtained. Of course, a nonspecific peptide with respect to the initial adhesion of bacteria, which does not prevent the effect, may be connected with one end or both ends of the above peptide comprising the specific amino acid sequence.

The composition for an oral cavity according to the present invention can be used with various embodiments according to the ordinary method. The composition may be used by only blending a peptide comprising a specific amino acid sequence with water or the other solvent being safe for a human body. This composition for an oral cavity may also be used for tooth powder, toothpaste, a liquid dentifrice or the like. The other blending ingredients is not limited if it does not prevent the function of the peptide, and the ingredients conventionally used in the composition for an oral cavity can be blended freely in general. More particularly, in the case of the toothpaste, abrasives, caking additives, wetting agents, sweeteners, perfumes, antiseptics, other pharmaceutically effective agents or the like can be suitably used.

More particularly, in the case of the tooth powder, the peptide comprising the specific amino acid sequence used in the present invention may be blended to the abrasives, such as calcium carbonate, calcium silicate, a silica fine powder, amorphous water-containing silica, hydrophobic silica, calcium secondary phosphate, calcium pyrophosphate, insoluble sodium metaphosphate, aluminum hydroxide, silicic acid anhydride, or the like. In the case of the toothpaste, the peptide comprising the specific amino acid sequence used in the present invention may be blended using the followings as a base material in general, that is, water, glycerin, ethylene glycol, diethylene glycol, polyethylene glycol, propylene glycol, polypropylene glycol, sorbitol, xylite, lactite, mannitol, ethanol, sodium lactate or the like. To the base material, the abrasives used to the tooth powder may be blended if necessary.

Further, various kinds of a surfactant, such as an anionic surfactant, a nonionic surfactant, an amphoteric surfactant, a semipolar surfactant or the like, and a thickener may be suitably blended. Examples of the anionic surfactant are an α-sulfofatty acid alkyl ester or its water-soluble salt, an alkyl sulfate ester salt, an N- acylamino acid salt, or the like. Examples of the nonionic surfactant are a fatty acid monoglyceride, a fatty acid alkanol amide, a polyoxyethylene sorbitan fatty acid ester, a polyoxyethylene hardened castor oil, a saccharide fatty acid ester, or the like. As the amphoteric surfactant, an alkylbetaine, imidazolinium betaine, a sulfobetaine, or the like are used. As the semipolar surfactant, an alkylamine oxide or the like is used. Examples of the thickener are sodium alginate, propylene glycol alginate ester, carboxymethyl cellulose, carboxymethylcellulose sodium, carboxy methylcellulose calcium, starch sodium glycolate, starch phosphate ester sodium, sodium polyacrylate, carboxypolymethylene, methylcellulose, crystalline cellulose, hydroxypropylcellulose, polyvinyl pyrrolidone, guar gum, carob bean gum, Tara gum, tamarind seed gum, gum Arabic, tragacanth gum, karaya gum, alginic acid, carrageenan, xanthan gum, gellan gum, curdlan, lactose, chitin, chitosan, chitosamine, or the like.

Further, a germicide, an antiinflammatory agent, a phosphoric acid compound and inorganic salts may be blended as pharmaceutically effective ingredients. Examples of the germicide are sodium monofluorophosphate, sodium fluoride, chlorhexidine salts, benzethonium chloride, cetylpyridinium chloride, benzalkonium chloride, or the like. As the antiinflammatory agent, allantoinate or the like is used. Examples of the phosphoric acid compound are sodium phosphate, sodium pyrophosphate, sodium tripolyphosphate, sodium tetrapolyphosphate, or the like. As the inorganic salts, sodium chloride or the like is used. Of course, a coloringmatter, a preservative or the like may be contained.

The composition for an oral cavity according to the present invention can be used as dental cleaning agents or medicines other than the above-mentioned kinds of tooth powder and toothpaste, such as a mouthwash, a chewing gum, a gargle, a troche, a cream, an ointment, a pasting agent and a tablet.

As for the peptide as defined above, the blending amount is 0.001-20 % by weight preferably, and more preferably 0.01-5 % by weight of the composition. In the case of the medicine, the amounts of peptide per adult per day in the case of an internal use is 5-50 mg, and the amounts in the case of an external use is 1 to several 10 mg a time.

Then, the peptide can be used for preventing the dental caries or periodontal disease without giving harmful side effects. In the case of the tooth powder, the toothpaste or the like, the peptide can be used according to the ordinary method, considering these usage amounts.

[Example]

Hereinafter, the composition for an oral cavity according to the present invention is explained with examples more concretely. However, the present invention is not limited to the following examples.

<Example 1>

<<Synthesis of the peptide>>

A peptide comprising an amino acid sequence of SSP-5(390-402) was obtained by a stepwise solid-phase peptide synthetic method. The synthesis was carried out with a reversed phase HPLC (0.1 % TFA of gradient of 10-45 % acetonitrile) with a TSK-GEL column (30 X1), by using Model 350 Multiple Peptide Synthesizer (Advanced Chemitech, Louisville, KY, USA) as a synthesizer. It was confirmed that a final purification degree is 95 % or more, by using the reversed phase HPLC.

<<Confirmation of adhesion suppressing properties>>

<Preparation of bacteria>

S. sanguis (ATCC10556, ATCC10558), S. mutans (ATCC25175) was used as intraoral bacteria. All bacteria were cultured under an anaerobic condition by using Brain Heart Infusion broth (BHI; Difco Lavoratory, Detroit, MI) as a culture medium.

<Preparation of saliva>

Stimulus saliva secreted by biting paraffine wax for 3 minutes by 3 subjects (A, B, C) was a specimen. The saliva was collected, kept quietly at 4°C for 5 minutes, and centrifuged at 10000 $\times$ g for 10 minutes at 4°C. Then, the supernatant was taken as the saliva specimen.

<Sensor chip>

The adhesion suppressing properties of bacteria was confirmed by using a sensor chip, which was a substitution of the tooth. As the sensor chip, Pioneer Sensor Chip F1 (produced by BIAcore Company) was used. The sensor chip F1 was activated at flow speed of 10 $\mu$L for 1 minute with 70 $\mu$L of 400mM N-ethyl- N'-(3-diaethylaminoprophyl) carbodiimide and 100mM N-hydroxy succinimide solution. Then, the above saliva specimen was diluted 20 times, and bonded on the chip with 70 $\mu$L. (Hereinafter, this chip was said to as a saliva treatment sensor chip)

<Confirmation method of the adhesion suppressing properties>

The confirmation method of the adhesion suppressing properties by BIA core™ Biosensor system (produced by BIAcore Company) will be explained. First, each bacteria flew at 0. D.=1 (550nm) and flow speed of 20 $\mu$L/min to the saliva treatment sensor chip. Then, the bonding state (Response Unit:RU) between the bacteria and saliva on the surface of the sensor chip was measured. The sensor chip was not carried out the peptide treatment.
Then, to the other saliva treatment sensor chip, which was different from the saliva treatment sensor chip adhered with bacteria, a SSP-5(390-402) peptide solution (1 mg/mL) dissolved with a phosphate buffer solution (PBS, pH7.4) flew on the sensor chip at flow speed of 10 $\mu$L/min, and the surface of the saliva treatment sensor chip was treated with the peptide. Then, each bacteria flew at O.D.=1 (550nm) and flow speed of 20 $\mu$L/min, and the bonding state (Response Unit:RU) between each bacteria and saliva on the sensor surface was measured. As for these results, the final bacterium adhesion suppressing ratio by the peptide treatment was measured using the following formula 1.

```
[Formula 1]

Adhesion suppressing ratio = 100 × {[Bonding state
between bacteria and saliva (RU)] – [Bonding state
between bacteria and saliva (after the peptide
treatment) (RU)]} / [Bonding state between bacteria
and saliva (RU)]
```

[Table 1]

| Adhesion suppressing ratio | | | |
|---|---|---|---|
| Saliva sample | S. sanguis (ATCC10556) | S. sanguis (ATCC10558) | S. mutans (ATCC25175) |
| A | 61% | 59% | 55% |
| B | 57% | 63% | 60% |
| C | 65% | 61% | 58% |

<Example 2>

As the composition for an oral cavity containing the peptide comprising the specific amino sequence according to the present invention, the toothpaste was prepared with following compositions. The adhesion suppressing properties of bacterial plaque is evaluated by the experiment described after.

| | |
|---|---|
| Calcium carbonate | 40 % by weight |
| Sodium carboxymethyl cellulose | 1.0 % by weight |
| Glycerin | 8.0 % by weight |
| Sodium lauryl sulfate | 1.5 % by weight |
| Sorbitol | 10.0 % by weight |
| Menthol | 0.3 % by weight |
| Peptide comprising a specific amino sequence | 1.0 % by weight |
| Water | 38.2 % by weight |

<Example 3>

As the composition for an oral cavity containing the peptide comprising the specific amino sequence according to the present invention, a tablet-shaped composition for an oral cavity was prepared with following compositions. The adhesion suppressing properties of bacterial plaque is evaluated by the experiment described after.

| | |
|---|---|
| Lactose | 65 % by weight |
| Crystalline cellulose | 26 % by weight |
| Magnesium stearate (lubricant) | 4 % by weight |
| Xylitol | 3 % by weight |
| Peptide comprising a specific amino sequence | 2 % by weight |

The effect of the composition for an oral cavity of Example 3 was confirmed by the following method. The method comprising, washing and polishing of the teeth surfaces of 3 subjects (A, B, C) in the dental clinic, brushing of only the left side teeth in an oral cavity of each 3 subjects after 3 time meals per day by using the toothpaste of Example 2, brushing of the right side teeth using a toothpaste, in which water was blended instead of the peptide comprising the specific amino sequence of Example 2, measuring of bacterial plaques of left and right side teeth with eyes by using a dyeing method with a dye for an oral cavity (PROSPEC GEL, produced by GC Corporation) after one weak, and comparing of these bacterial plaques. These results were shown in Table 2 collectively.

[Table 2]

| Subjects | Left side in an oral cavity | Right side in an oral cavity |
|---|---|---|
| A | Bacterial plaque is Bacterial plaque is hardly dyed | Bacterial plaque is colored red a little is colored red a little and can be confirmed |
| B | Bacterial plaque is Bacterial plaque is hardly dyed | Bacterial plaque is colored red a little is colored red a little and can be confirmed |
| C | Bacterial plaque is Bacterial plaque is hardly dyed | Bacterial plaque is colored red a little and colored red a little and can be confirmed |

The effect of the composition for an oral cavity of Example 3 was confirmed by the following method. The method comprising, washing and polishing of the teeth surfaces of 3 subjects (D, E, F) in the dental clinic, brushing of the teeth of each 3 subjects 3 times per day without using the toothpaste, and confirming of the amounts of bacterial plaques by the dyeing method after one weak like the above-mentioned method. Then, the evaluation was continued by the method comprising, washing and polishing of the teeth surfaces of 3 subjects in the dental clinic again, brushing of the teeth after 3 time meals per day without using the toothpaste, eating the tablet-shaped composition for an oral cavity of Example 3 3 times per day, and confirming of the amounts of bacterial plaques by the dyeing method with the dye for an oral cavity (PROSPEC GEL, produced by GC Corpotation) after one weak. These results were shown in Table 3 collectively.

[Table 3]

| Subjects | Composition for an oral cavity was not used | Composition for an oral cavity was used |
|---|---|---|
| D | Bacterial plaque is colored red a little and little and can be confirmed | Bacterial plaque is hardly Bacterial plaque is hardly dyed |
| E | Bacterial plaque is colored red a little and can be confirmed | Bacterial plaque is hardly dyed |
| F | Bacterial plaque is colored red a little and can be confirmed | Bacterial plaque is hardly dyed |

**Claims**

1. A composition for the oral cavity containing a peptide of the amino acid sequence Asp Tyr Gln Ala Lys Leu Ala Ala Tyr Gln Lys Glu Leu.

2. The composition according to Claim 1, wherein the amount of peptide is 0.001-20 % by weight of the composition.

**Patentansprüche**

1. Zusammensetzung für die Mundhöhle, enthaltend ein Peptid der Aminosäuresequenz Asp Tyr Gln Ala Lys Leu Ala Ala Tyr Gln Lys Glu Leu.

2. Zusammensetzung gemäß Anspruch 1, wobei die Menge des Peptids 0,001-20 Gew.-% der Zusammensetzung beträgt.

**Revendications**

1. Composition pour la cavité orale contenant un peptide d'une séquence d'acides aminés Asp Tyr Gln Ala Lys Leu Ala Ala Tyr Gln Lys Glu Leu.

2. Composition selon la revendication 1, dans laquelle la quantité de peptide est de 0,001 à 20% en poids de la composition.